# EUROPEAN PATENT APPLICATION

(11) **EP 4 497 439 A1**
(43) Date of publication of application: **29.01.2025**
(21) Application number: 23382792.2
(22) Date of filing: 28.07.2023
(51) Int. Cl.: A61K 31/522, A61K 31/165, A61K 31/195, A61K 31/343, A61K 31/4152, A61K 31/4196, A61K 31/426, A61K 31/519, A61P 19/02, A61P 19/06

(54) **COMPOUNDS FOR THE TREATMENT OR PREVENTION OF GOUT**

(71) Applicant: Universitat de les Illes Balears, 07122 Palma de Mallorca (ES)
(72) Inventor: COSTA BAUZÀ, Antonia, 07122 PALMA DE MALLORCA (ES); GRASES FREIXEDAS, Felix, 07122 PALMA DE MALLORCA (ES)
(74) Representative: ZBM Patents - Zea, Barlocci & Markvardsen

(57) **Abstract**

The present invention provides compound of formula (I) or a pharmaceutically acceptable salt thereof, for use in the prevention and/or treatment of a disease or disorder associated with the crystallization of monosodium urate monohydrate, wherein R₁ and R₂ are different and are selected from -H and -(C₁-C₆)alkyl; and R₃ is selected from -H and =O. The invention also provides an in vitro method for eliminating and/or preventing the formation of monosodium urate monohydrate crystals in a composition, and an i*n vitro* use of a compound of formula (I) for eliminating and/or preventing the formation of monosodium urate monohydrate crystals.

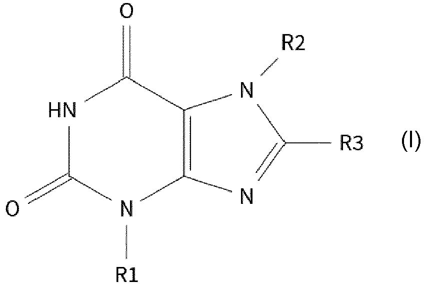

## Description

### Technical Field

The present invention belongs to the field of compounds capable of preventing the crystallization of monosodium urate monohydrate. The compounds of the invention are particularly useful as a medicament for preventing or treating gout.

### Background Art

Gout is a rheumatic pathology that affects the joints, mainly the feet, although it often also affects the hands. Gout causes severe pain and swelling, and it is originated by the deposition of needle-shaped monosodium urate (MSU) crystals. These crystals should not be confused with the uric acid (UA) crystals that cause renal lithiasis, which have a different structure and etiology.

Uric acid is a product resulting from purine metabolism, and at pH values of 7.4, such as those found in plasma and interstitial fluids, it is found in the ionic form of urate, which combines with sodium ions to form MSU. Under normal conditions, the amount of uric acid present in the blood does not exceed 6 mg/dL. For these concentrations, MSU is soluble and does not generate any type of crystal. However, there are people who, due to genetic aspects related to the synthesis of uric acid or the consumption of diets especially rich in purines (e.g. red meat, viscera, sausages, fish, and shellfish) and the consumption of alcoholic beverages, generate significant amounts of uric acid, which exceeds 6 mg/dL, which can even reach concentrations of 20 mg/dL.

For uric acid concentration values greater than 6 mg/dL, the solubility of MSU in interstitial fluids is exceeded, which is why crystallization of this salt can take place in the form of fine needles of MSU, which are the ones that cause the painful episodes of gout.

At the present time, therapeutic strategies for preventing the formation of MSU crystals are mainly based on strict diets and the use of Xanthine oxidoreductase (XOR) inhibitors, such as Allopurinol and Febuxostat, to reduce serum -and, therefore, synovial fluid-urate levels. However, these approaches are frequently ineffective and XOR-inhibitor drugs often have significant adverse effects.

Therefore, in spite of the efforts made so far, there is still a need for compounds capable of inhibiting the formation of MSU crystals useful in the treatment of gout.

### Summary of Invention

The present inventors have discovered a particular group of compounds that has a strong and specific capacity to prevent the formation of MSU crystals.

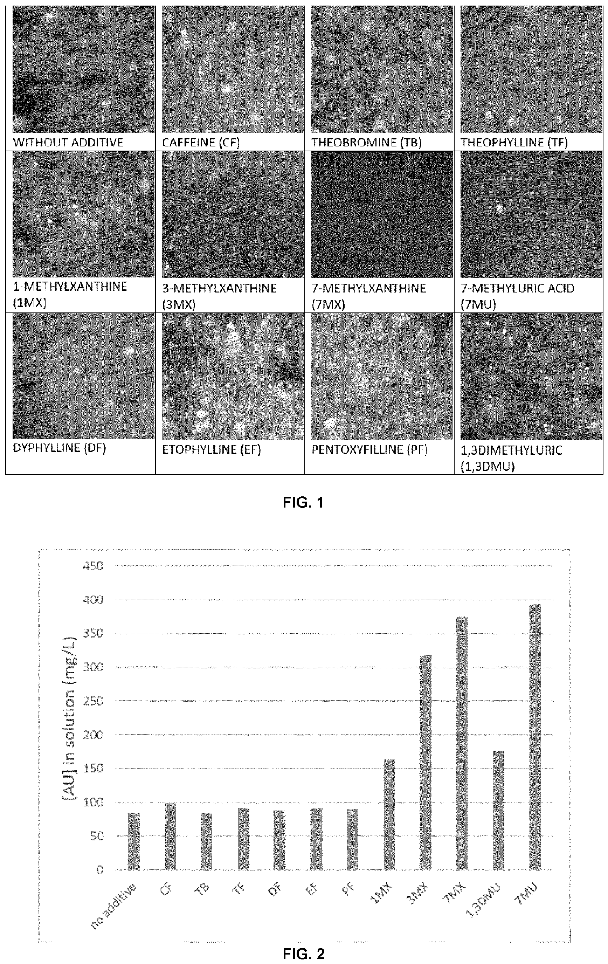

As shown in the examples below, the inventors have found that the methylxanthines of formula (I) are capable of inhibiting the formation of MSU crystals (see Figure 1 and 2). This was highly unexpected as structurally related methylxanthines -such as theobromine, which is known to prevent the formation of uric acid (UA) crystals- did not show any effect on MSU crystallization (see Figure 2).

Furthermore, compounds of formula (I) have been previously used for the treatment of other diseases in humans, such as myopia, without any reported side-effects, therefore, they constitute a safe therapeutic approach.

In conclusion, from the experimental data provided herein it is apparent that the compounds of the invention can be used as a safe and efficient medicament for treating human diseases associated with MSU crystallization, such as gout.

Thus, in a first aspect, the invention provides a compound of formula (I) 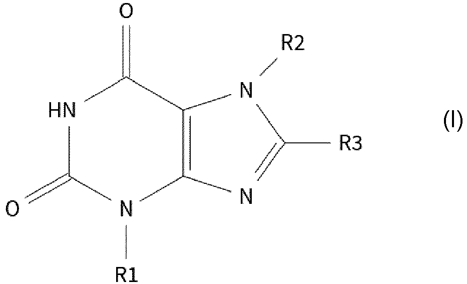 or a pharmaceutically acceptable salt thereof, for use in the prevention and/or treatment of a disease or disorder associated with the crystallization of monosodium urate monohydrate, wherein R₁ and R₂ are different and are selected from -H and -(C₁-C₆)alkyl; and R₃ is selected from -H and =O.

In a second aspect, the present invention provides an *in vitro* method for eliminating and/or preventing the formation of monosodium urate monohydrate crystals in a composition comprising uric acid, the method comprising contacting the composition with a compound of formula (I) 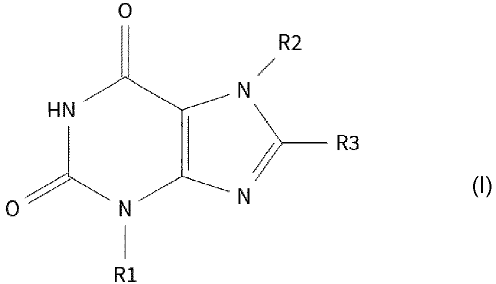 or a pharmaceutically acceptable salt thereof, wherein R₁ and R₂ are different and are selected from -H and -(C₁-C₆)alkyl; and R₃ is selected from -H and =O.

In a third aspect, the present invention provides the *in vitro* use of a compound of formula (I) 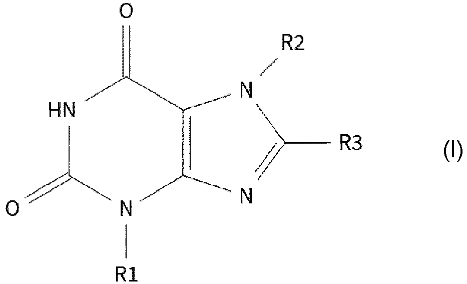 or a pharmaceutically acceptable salt thereof, for eliminating and/or preventing the formation of monosodium urate monohydrate crystals, wherein R₁ and R₂ are different and are selected from -H and -(C₁-C₆)alkyl; and R₃ is selected from -H and =O.

### Brief Description of Drawings

Figure 1. Twelve-wells plate images after 6 days (144 h) of incubation. Each well had an initial concentration of uric acid of 375 mg/L (2.23 mM), [Na+] = 400 mM and phosphate buffer 12 mM at pH = 7.4. Tested compounds were added at a concentration of 0.3 mM.
Figure 2. Concentration of uric acid that remained in solution after 4 days of incubation in presence of each compound. Each well had an initial concentration of uric acid of 375 mg/L (2.23 mM), [Na+] = 400 mM and phosphate buffer 12 mM at pH = 7.4. Tested compounds were added at a concentration of 0,3 mM.
Figure 3. Twelve-wells plate image after 3 days (72 h) of incubation. Each well contained a solution with [UA] = 375 mg/L (2.23 mM), [Na+] = 400 mM and phosphate buffer 12 mM at pH = 7.4. Three wells of first column contained no additive, three wells of second column contained 50 µM (8.3 mg/L) of 7-methylxanthine, three wells of third column contained 100 µM (16.3 mg/L) of 7-methylxanthine and three wells of fourth column contained 150 µM (24.9 mg/L) of 7-methylxanthine.
Figure 4. Concentration of uric acid that remained in solution after 5 days (120 h) of incubation in presence of different concentrations of 7-methylxanthine or 3-methylxanthine. Each well had an initial concentration of uric acid of 375 mg/L (2.23 mM), [Na+] = 400 mM and phosphate buffer 12 mM at pH = 7.4.
Figure 5. Concentration of uric acid that remained in solution after 5 days (120 h) of incubation in presence of different mixtures of 7-methylxanthine and 3- methylxanthine. Each well had an initial concentration of uric acid of 375 mg/L (2.23 mM), [Na+] = 400 mM and phosphate buffer 12 mM at pH = 7.4.
Figure 6. Concentration of uric acid that remained in solution after 3 days (72 h) of incubation in presence of different concentrations of uric acid and in function of 7-methylxanthine. Each well had an initial concentration of [Na+] = 400 mM and phosphate buffer 12 mM at pH = 7.4.
Figure 7. Concentration of uric acid that remained in solution after 4 days (96 h) of incubation in presence of different concentrations of uric acid and in function of 7-methylxanthine. Each well had an initial concentration of [Na+] = 400 mM and phosphate buffer 12 mM at pH = 7.4.
Figure 8. Photographs of the 12-well plates containing different initial concentrations of uric acid in absence and presence of different levels of 7-methylxanthine after 120 h from the beginning of the experiment.
Figure 9. Concentration of uric acid that remained in solution after 5 days (120 h) of incubation in presence of different concentrations of uric acid and in function of 7-methylxanthine. Each well had an initial concentration of [Na+] = 400 mM and phosphate buffer 12 mM at pH = 7.4.
Figure 10. Minimum concentration of 7-methylxanthine that totally avoided the formation of sodium urate crystals (C100) in presence of different initial concentrations of uric acid. The solution also contained [Na+] = 400 mM and phosphate buffer 12 mM at pH = 7.4.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

For purposes of the present invention, any ranges given include both the lower and the upper end-points of the range. Ranges given, such as concentrations and the like, should be considered approximate, unless specifically stated.

As above described, in a first aspect the present invention provides a compound of formula (I) 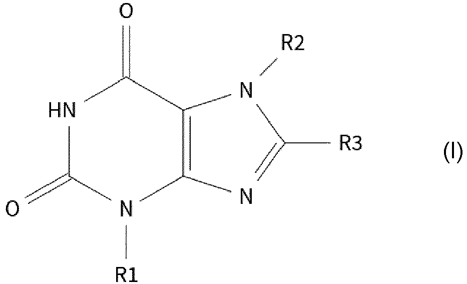 or a pharmaceutically acceptable salt thereof, for use in the prevention and/or treatment of a disease or disorder associated with the crystallization of monosodium urate monohydrate, wherein R₁ and R₂ are different and are selected from -H and -(C₁-C₆)alkyl; and R₃ is selected from -H and =O.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutical acceptable salts are well known in the art. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, trifluoroacetic acid, oxalic acid, maleic acid, tartaric acid, citric acid, succinic acid or malonic acid or by using other methods used in the art such as ion exchange. Other pharmaceutical acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, and ammonium. Representative alkali or alkaline earth metal salts include sodium, lithium, potassium, calcium, magnesium, and the like. Further pharmaceutical acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate.

The skilled person is able to prepare pharmaceutically acceptable salts of the compounds of formula (I), in particular of 7MX, following routine methods.

As used herein, "a disease or disorder associated with the crystallization of monosodium urate monohydrate" refers to any disease or disorder characterized by the presence of detrimental monosodium urate monohydrate (MSU) crystals. The terms "monosodium urate monohydrate", "monosodium urate", "sodium urate", and "MSU" are used interchangeably in the present application, and refer to the compound of formula (NaC₅H₃N₄O₃·H₂O), in which a urate molecule is bonded to one sodium and one water molecule. A "disease" is a state of health of an animal wherein the animal cannot maintain homeostasis, and wherein if the disease is not ameliorated then the animal's health continues to deteriorate. In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.

The term "treatment", as used herein, unless otherwise indicated, refers to any type of therapy which is aimed at terminating, preventing, ameliorating or reducing the susceptibility to a clinical condition or existing disease as described herein, including complete curing of a disease as well as amelioration or alleviation of said disease. Thus, "treatment," "treating," and their equivalent terms refer to obtaining a desired pharmacologic or physiologic effect, covering any treatment of a pathological condition or disorder in a subject. The term "prevention" or "preventing", as used herein, means, but is not limited to, a process of prophylaxis in which a subject is exposed to the compound of the invention prior to the induction or onset of the disease process.

The term "(C₁-C₆)alkyl" refers to linear or branched saturated hydrocarbon chains that have 1 to 6 carbon atoms, for example, methyl, ethyl, n-propyl, i-propyl, n-butyl, tert-butyl, sec-butyl, n-pentyl, n-hexyl, etc. more particularly of 1 to 3 carbon atoms, and more particularly it is a methyl.

In one embodiment, the disease or disorder associated with the crystallization of monosodium urate monohydrate is gout.

The term "gout", as used herein, refers to a systemic disease that results from the deposition of monosodium urate crystals (MSU) in tissues, particularly in the joints. The joint can include, but is not limited to, the right or left metatarsophalangeal joint (MTP), ankle, instep, wrist, shoulder, hip, knee, elbow, metacarpal phalangeal (MCP), interphalangeal (IP), proximal inter-phalangeal (PIP), or distal inter-phalangeal (DIP) joints. There are a number of stages of gout. For example, patients or subjects suffering from or having "early gout" as used herein, are those patients that are hyperuricemic and that have experienced or have a history of only a single gout flare in a joint during said subject's lifetime. A gout flare is an "attack" or exacerbation of gout symptoms, which can happen at any time. Patients or subjects suffering from or having "later stage gout" as used herein, are those patients that are hyperuricemic and that have experienced or have a history of more than one (or multiple) gout flares in a joint during said subject's lifetime. The term "gout" as used herein refers to both "early gout" and "later stage gout" collectively.

In one embodiment, R₁ and R₂ are different and are selected from -H and -(C₁-C₃)alkyl. In a more particular embodiment, R₁ and R₂ are different and are selected from -H and -CH₃.

In one embodiment, R₁ and R₂ are different and are selected from -H and -CH₃, and R₃ is - H. In another embodiment, R₁ is -H, R₂ is -CH₃, and R₃ is -H. In another embodiment, R₁ is -CH₃, R₂ is -H, and R₃ is -H.

In one embodiment, the compound is selected from the group consisting of 7-methylxanthine, 3-methylxanthine, 3-methyluric acid, 7-methyluric acid, and mixtures thereof. In a more particular embodiment, the compound is selected from the group consisting of 7-methylxanthine, 3-methylxanthine, 7-methyluric acid, and mixtures thereof. In a more particular embodiment, the compound is selected from 7-methylxanthine and 3-methylxanthine. In an even more particular embodiment, the compound is 7-methylxanthine.

7-Methylxanthine or 7MX (C₆H₆N₄O₂) has the CAS number 552-62-5; 3-methylxanthine or 3MX (C₆H₆N₄O₂) has the CAS number 1076-22-8; 7-methyluric acid or 7MU (C₆H₆N₄O₃) has the CAS number 612-37-3; and 3-methyluric acid or 3MU (C₆H₆N₄O₃) has the CAS number 605-99-2. All these compounds can be synthesized following routine methods known in the art and are commercially available from different companies, for example, 7-methylxanthine can be purchased from Sigma Aldrich (ref. 69723).

In one embodiment, the compound is for use in the prevention and/or treatment of a disease or disorder associated with the crystallization of monosodium urate monohydrate, particularly gout, in a subject. As used herein, the term "subject" refers to an animal, preferably a mammal, including a human or non-human. The terms patient and subject may be used interchangeably herein.

In one embodiment, the compound is for use in a subject that is hyperuricemic. As used herein, "hyperuricemic" refers to a subject that has a serum urate or serum uric acid level greater than or equal to 7.0 mg/dL. The skilled person would know how to measure serum urate or serum uric acid levels following routine methods known in the art, such as those disclosed in the examples below.

In one embodiment, the compound of formula (I) is for use in the prevention and/or treatment of a disease or disorder associated with the crystallization of monosodium urate monohydrate, particularly gout, by inhibiting the crystallization of monosodium urate monohydrate and/or increasing the solubility of uric acid. In another embodiment, the compound of formula (I) is for use in the prevention and/or treatment of a disease or disorder associated with the crystallization of monosodium urate monohydrate, particularly gout, by eliminating and/or preventing the formation of monosodium urate monohydrate crystals.

In one embodiment, the compound of formula (I) or the pharmaceutically acceptable salt thereof is administered in a dose from 1 mg to 4000 mg, from 10 mg to 3000 mg, from 100 mg to 2000 mg, from 500 mg to 1500 mg, or from 800 mg to 1200 mg, per day. More particularly, it is administered in a dose from 500 mg to 1500 mg per day.

In one embodiment, the compound is comprised in pharmaceutical composition, a nutraceutical composition, a functional food, or a food supplement. In another embodiment, the compound is administered in the form of a pharmaceutical composition, a nutraceutical composition, a functional food, or a food supplement. In a more particular embodiment, the compound is comprised in a therapeutically effective amount in a pharmaceutical composition together with one or more pharmaceutically acceptable excipients and/or carriers. In a more particular embodiment, the compound is administered in a therapeutically effective amount in the form of a pharmaceutical composition together with one or more pharmaceutically acceptable excipients and/or carriers.

In the present invention, a "nutraceutical composition" or "functional food" is understood as a food that has a beneficial effect on health. Similarly, the term nutraceutical can be applied to extracts or chemical compounds obtained from common foods. Examples of foods that have been attributed nutraceutical properties are olive oil, red wine, broccoli, soybeans, etc. Nutraceuticals are normally used in nutritional mixtures and in the pharmaceutical industry. Just as some foods can be classified as nutraceuticals it is also possible to classify some nutritional supplements in the same way, such as, for example, fatty acids such as omega-3 derived from fish oil and from some vegetables or antioxidants and vitamins.

The expression "therapeutically effective amount", as used herein, refers to the amount of compound or pharmaceutically acceptable salt thereof that, when administered, is sufficient to prevent development of, or alleviate to some extent, one or more of the symptoms of the disease which is addressed. The particular dose of agent administered according to this invention will of course be determined by the particular circumstances surrounding the case, including the route of administration, the particular condition being treated, and similar considerations.

The expression "pharmaceutically acceptable excipients and/or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and non-human animals without excessive toxicity, irritation, allergic response, immunogenicity or other problems or complications commensurate with a reasonable benefit/risk ratio. Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The compound and the composition provided by the present invention can be administered by any suitable route, for example, oral, parenteral, topical, intranasal or sublingual route, for which they will include the acceptable excipients necessary for the formulation of the desired dosage form. In one embodiment, the compound or the composition is administered orally.

It is clear for the skilled person that the composition may be prepared using state of the art excipients and applying usual pharmaceutical technologies.

The administration of the compound with formula (I), and particularly 7MX, can be combined with one or more compounds that facilitate its absorption through a selected administration route. It can, therefore, be administered with lactose, sucrose, talc, magnesium stearate, cellulose, calcium salts, gelatine, fatty acids, as well as other similar substances.

For therapeutic use, it is preferable if the compound with formula (I), particularly 7MX, is in a pharmaceutically acceptable form or is substantially pure, that is, that is has a pharmaceutically acceptable level of purity excluding the normal pharmaceutical additives, such as diluents and carriers, and is free from any materials considered toxic at normal dosage levels. The purity levels for the active substance are particularly above 50%, more particularly above 70%, and still more particularly above 90%. In a particular embodiment, the levels of the compound with formula (I), or its salts, are above 95%.

The dosage form may be a solid pharmaceutical composition such as tablets or coated tablets, powders, fine granules, granules, capsules e.g. hard or soft gelatin capsules, troches (pastilles), a bolus and chewable preparations containing the compound of formula (I) or a pharmaceutically acceptable salt thereof.

Alternatively, the pharmaceutical composition may be a semisolid or liquid dosage form such as gel, e.g. a hydrogel, a cream, an ointment, a lotion, water-in-oil or oil-in-water emulsions, suspensions, aerosols, and liquid preparations such as solutions, elixirs, syrups including dry syrups.

The preparation of pharmaceutical forms of the above-mentioned kind is well-known *per* se from the prior art. The dose to be administered may appropriately be controlled depending on the dosage forms of the desired pharmaceutical preparations.

The pharmaceutical composition of the invention may be administered to a patient in a daily dose in portions over one or several times per day. The amount of the effective substance may also be formulated into a single dose, in as much as it is not unreasonable from the viewpoint of the dosage form of the pharmaceutical preparation.

In one embodiment, the solid dosage form such as a capsule, tablet, pastille, granule, a powder or a liquid or another dosage form for oral application may contain the compound of formula (I) or a pharmaceutically acceptable salt thereof in an amount allowing to provide from 1 mg to 4000 mg, from 10 mg to 3000 mg, from 100 mg to 2000 mg, from 500 mg to 1500 mg, or from 800 mg to 1200 mg of the active ingredient per single dose.

In the preparation of the composition comprises the compound of formula (I), a variety of currently used additives may be employed, such as one or more of a filler, a thickening agent, a gelling agent, a binder, a disintegrator, a surfactant, a lubricant, a coating agent, a sustained release agent, a diluent and/or one or more excipients. In addition to the foregoing, the agent of the present invention may, if necessary, further comprise other additives such as a solubilizing agent, a buffering agent, a preservative, an isotonic agent, an emulsifying agent, a suspending agent, a dispersant, a hardening agent, an absorbent, an adhesive, an elasticizing agent, an adsorbent, a perfume, a coloring agent, a corrigent, an antioxidant, a humectant, a light-screening agent, a brightener, a viscosity enhancer, an oil, a tableting adjuvant, and/or an anti-static agent.

More specifically, examples of such additives include one or more excipients such as lactose, corn starch, mannitol, D-sorbitol, crystalline cellulose, erythritol and sucrose; a binder such as hydroxypropyl cellulose (HPC-L), hydroxypropyl methyl cellulose, polyvinyl pyrrolidone, methyl cellulose and gelatinized starch; a disintegrator such as calcium carboxymethyl cellulose, crosslinked sodium carboxymethyl cellulose and crosslinked polyvinyl pyrrolidone (crospovidon); a lubricant such as magnesium stearate and talc; a perfume, for instance, a flavor or an aromatic oil such as apple essence, honey flavour, 1-menthol, vanillin, lemon oil, cinnamon oil, mentha oil or peppermint oil; and/or an adsorbent such as synthetic aluminum silicate and light anhydrous silicic acid.

Moreover, it is also possible to prepare coated pharmaceutical preparations through the use of a currently used coating agent such as hydroxypropyl methyl cellulose, hydroxypropyl cellulose, methyl cellulose or polyvinyl pyrrolidone.

If necessary, a sweetener may likewise be used, such as in troches, syrups and chewable preparations among others. Specific examples of such sweeteners are mannitol, glucose, maltose, starch syrup, malt extract, maltitol, sorbitol, sucrose, unrefined sugar, fructose, lactose, honey, xylitol, hydrangea tea, saccharin, aspartame, cyclamate, Sunett^{®}, aspartyl phenylalanine ester and other malto-oligo saccharides, and oligo saccharides such as maltosyl sucrose, isomaltyrose of reduced type and raffinose, Acesulfame potassium or any kind of sugar alcohols or mixtures thereof such as sorbitol, mannitol and/or xylitol.

As solubilisers any known solubiliser suitable in the medical sector may be used, for example polyethyleneglycols, polyoxyethylene-polyoxypropylene copolymers (e.g. poloxamer 188), glycofurol, arginine, lysine, castor oil, propyleneglycol, solketal, polysorbate, glycerol, polyvinyl pyrrolidone, lecithin, cholesterol, 12- hydroxystearic acid-PEG660-ester, propyleneglycol monostearate, polyoxy-40- hydrogenated castor oil, polyoxyl-10-oleyl-ether, polyoxyl-20-ceto-stearylether and polyoxyl-40-stearate or a mixture thereof.

Any preservatives known for use in the pharmaceutical field may be used, for example, ethanol, benzoic acid and the sodium or potassium salts thereof, sorbic acid and the sodium or potassium salts thereof, chlorobutanol, benzyl alcohol, phenylethanol, methyl-, ethyl-, propyl- or butyl-p-hydroxybenzoates, phenol, m-cresol, p-chloro-m-cresol, those selected from the group of the PHB esters, e.g. mixtures of PHB-methyl with PHB-propylesters, quaternary ammonium compounds such as benzalkonium chloride, thiomersal, phenyl-mercury salts such as nitrates, borates.

The buffer system used to achieve a desired pH value may be, for example, glycine, a mixture of glycine and HCl, a mixture of glycine and sodium hydroxide solution, and the sodium and potassium salts thereof, a mixture of potassium hydrogen phthalate and hydrochloric acid, a mixture of potassium hydrogen phthalate and sodium hydroxide solution or a mixture of glutamic acid and glutamate.

Suitable gelling agents are for example cellulose and its derivatives, like for instance methyl cellulose, carboxymethyl cellulose, hydroxypropylmethyl cellulose, poly(vinyl)alcohol, polyvinylpyrrolidones, polyacrylates, poloxamers, tragacanth, carrageenan, starch and its derivatives or any other gelling agent used in pharmaceutical technology.

Viscosity enhancers which may be mentioned are for example the aforementioned gelling agents in low quantities, glycerol, propylene glycole, polyethylene glycol or polyols, like sorbitol and other sugar alcohols.

The emulsifiers used, apart from the emulsifiers known from the prior art, may include polyoxyethylene derivatives of castor oil or polyoxyethylene alkylethers.

Suitable synthetic or natural, coloring agents known in the pharmaceutical field may be used such as Indigo carmine.

Suitable oily components which may be present are any of the oily substance known from the prior art for the preparation of pharmaceuticals, such as, for example, vegetable oils, in particular, e.g. cotton seed oil, groundnut oil, peanut oil, maize oil, rapeseed oil, sesame oil and soya oil, or triglycerides of moderate chain length, e.g. fractionated coconut oil, or isopropylmyristate, -palmitate or mineral oils or ethyloleate.

The antioxidants used may be any of the antioxidants known from the prior art, for example a-tocopherol, butylhydroxytoluene (BHT) or butylhydroxyanisole (BHA).

Pharmaceutical compositions containing these additives may be prepared according to any method known in this field, depending on the dosage form. It is a matter of course that further additives not explicitly discussed may be used in the formulations used according to the present invention.

In one embodiment, the pharmaceutical composition is for immediate release of the compound of formula (I) or a pharmaceutically acceptable salt thereof. These types of formulations allow a quick release of the active ingredient serving for the purpose of rapidly acting on the different tissues. On the alternative and in another particular embodiment, the pharmaceutical composition is a composition for the controlled along time release of the active principle, also termed modified-release composition or dosage forms. In particular, it is for the release of the active principle from 1 to 2 days. More particularly, it is for the release of the active principle from 1 to 2 days and is formulated for administration to a subject up to once a day or once every two days. These compositions allow reducing the administration times and promote a simpler posology. Examples of these non-immediate release compositions, in which active principle is released during a period of time, comprise sustained-release compositions and prolonged release compositions. Sustained-release dosage forms are dosage forms designed to release (liberate) a drug at a predetermined rate in order to maintain a constant drug concentration for a specific period of time with minimum side effects. This can be achieved through a variety of formulations, including drug-polymer conjugates. Prolonged release compositions are compositions in which the drug is delivered within a period of time (extended-release usually at a constant rate) or to a specific target in the body (targeted-release dosage).

In one embodiment, the compound of formula (I) or a pharmaceutically acceptable salt thereof is administered in the form of a metabolic precursor.

As used herein, "metabolic precursor" refers to a compound that is metabolized by a tissue or enzyme of the subject to the compound of formula (I), in particular 7MX, for example theobromine or caffeine (Arnaud MJ et al., "Pharmacokinetics and Metabolism of Natural Methylxanthines in Animal and Man", Methylxanthines. Handbook of Experimental Pharmacology, vol. 200). Thus, in one embodiment, the metabolic precursor is theobromine or caffeine.

In one embodiment, the compound of formula (I) or a pharmaceutically acceptable salt thereof is administered in the form of a composition comprising a metabolic precursor, in particular coffee or chocolate.

In one embodiment, the compound is administered in combination with a compound selected from the group consisting of colchicine, a compound that inhibits uric acid synthesis, a compound that promotes uric acid excretion, a non-steroidal anti-inflammatory agent, a corticoid, and mixtures thereof. In a more particular embodiment, the compound that inhibits uric acid synthesis is Allopurinol or Febuxostat, and/or the compound that promotes uric acid excretion is selected from the group consisting of Probenecid, Sulfinpyrazone, Benzbromarone and Lesinurad.

All embodiments above described for the first aspect are also meant to apply to the second and third aspects of the invention.

In a particular embodiment of the second and third aspects, the compound is 7-methylxanthine.

In a particular embodiment of the second and third aspects, the disease or disorder associated with the crystallization of monosodium urate is gout.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

The effects of different methylxanthines on the crystallization of monosodium urate monohydrate (MSU) were studied in an *in vitro* model. In particular, it was evaluated whether MSU crystallization could be inhibited by:
- Caffeine (1,3,7-trimethylxanthine) (CF)
- Theobromine (3,7-dimethylxanthine) (TB)
- Theophylline (1,3-dimethylxanthine) (TF)
- 1-methylxanthine (1MX)
- 3-methylxanthine (3MX)
- 7-methylxanthine (7MX)
- Dyphylline (DF)
- Etophylline (EF)
- Pentoxyfilline (PF)
- 1,3-dimethyluric acid (1,3DMU)
- 7-methyluric acid (7MU)

Each methylxanthine was added at a concentration of 0.3 mM in wells containing uric acid at a concentration of 375 mg/L (2.23 mM), Na⁺ at a concentration of 400 mM, and phosphate buffer at a concentration of 12 mM and at pH = 7.4. Plates were incubated for 6 days (144h) and the formation of MSU crystals was then analyzed.

Formation of MSU crystals was evaluated by naked eye observation and by determination of uric acid concentration that remained in solution, which was measured with an adapted version of the phosphotungstic acid method. Briefly, each sample, adequately diluted, was mixed with phosphotungstic acid and sodium carbonate solutions, and then incubated at 25°C for 25 min. The optical density of the colored product, tungsten blue, was read at 700 nm using a 96-wells plate reader and compared with a calibration graph.

As shown in Figures 1 and 2, the majority of the methylxanthines tested did not exert any significant effect on the crystallization of MSU. However, 7-methylxanthine, 3-methylxanthine and 7-methyluric acid surprisingly exerted a strong inhibition in the formation of these crystals. This effect was not only important in inhibiting crystallization, but also significantly increased the solubility of sodium urate in the tested fluid. Without wishing to be bound to any theory, this increase in solubility may be attributable to the formation of stable "clusters" between urate ions and 7-methylxanthine, 3-methylxantine or 7-methyluric acid, in the liquid medium, thus decreasing the real supersaturation of sodium urate in blood. It may therefore be mainly a thermodynamic effect and not kinetic in nature.

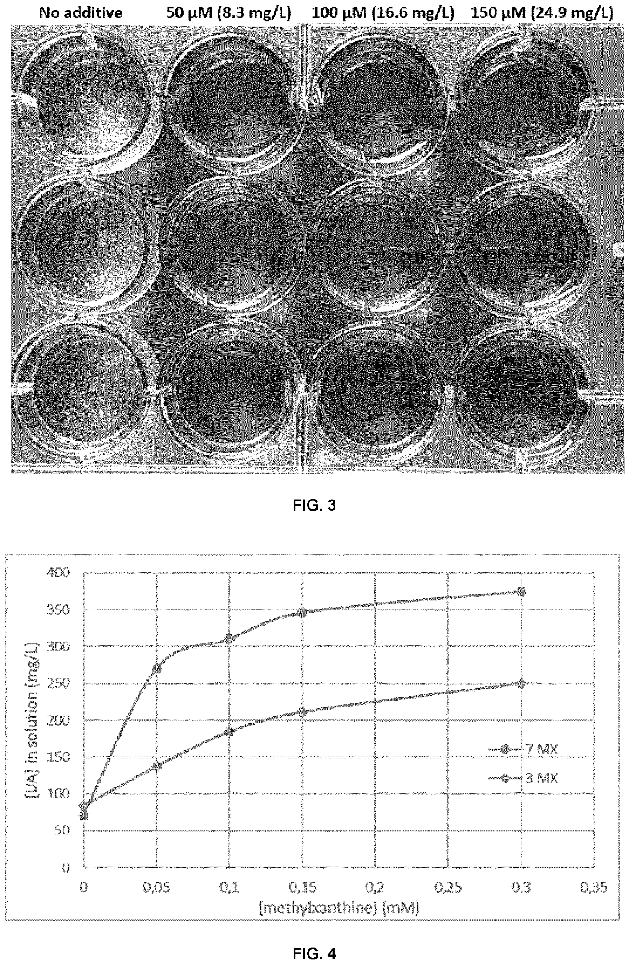

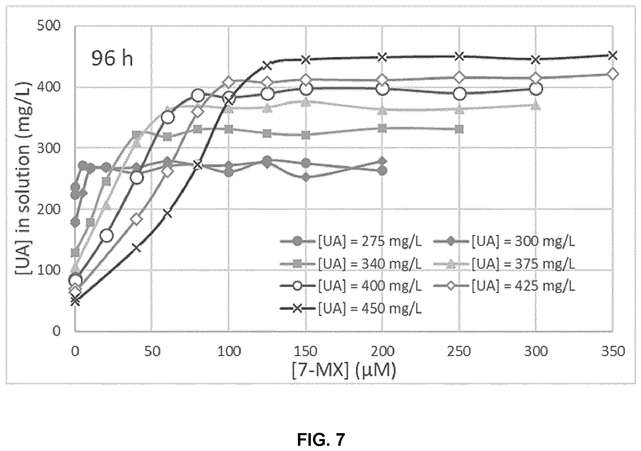

Then, the effect of 7-methylxanthine on MSU crystal formation was tested at various concentrations -50 µM, 100 µM and 150 µM- in triplicates. As shown in Figure 3, 7-methylxanthine completely prevented the formation of MSU crystals after 3 days at all concentrations tested.

Next, the inhibiting effects of 7-methylxanthine and 3-methylxanthine were compared at different concentrations for 5 days. As shown in Figure 4, both 7-methylxanthine and 3-methylxanthine were capable of preventing the formation of MSU crystals when added at concentrations as low as 50 µM, as evidenced by an increased presence of uric acid in solution. However, 7-methylxanthine showed a significantly higher crystallization inhibition compared to 3-methylxanthine.

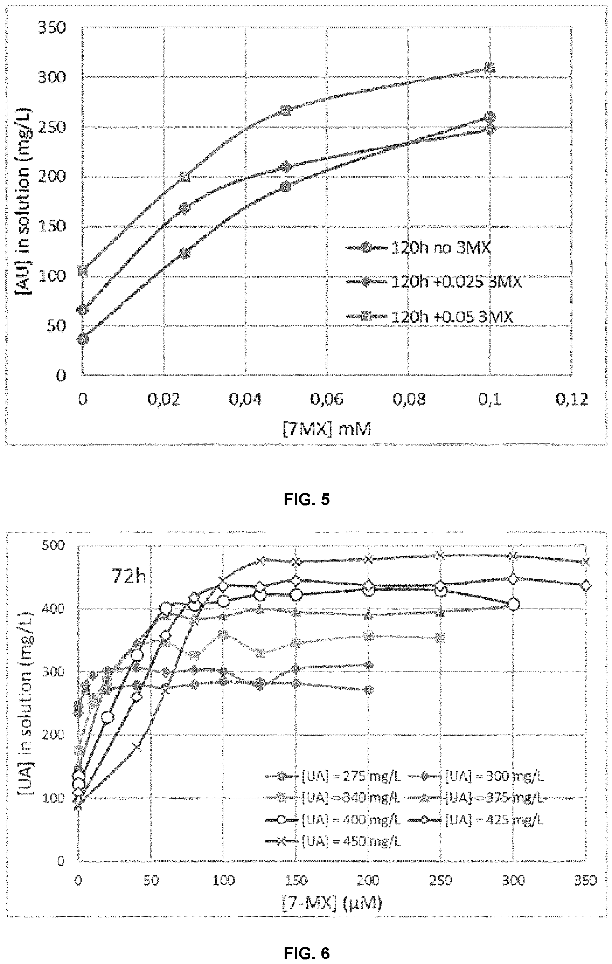

Then, it was tested the effect on MSU crystallization of the combination of 7-methylxanthine and 3- methylxanthine. As shown in Figure 5, the combination 7-methylxanthine and 3- methylxanthine further prevented the formation of MSU crystals.

It was also studied the minimal concentration of 7-methylxanthine needed to completely inhibit MSU crystallization at different concentrations of uric acid. The conditions tested are shown in Table 1:

**Table 1**

| [AU] (mg/L) | [7-mentylxanthine] (µM) | | | | | | | | | | | |
|---|---|---|---|---|---|---|---|---|---|---|---|---|
| 275 | 0 | 0 | 5 | 10 | 20 | 40 | 60 | 80 | 100 | 125 | 150 | 200 |
| 300 | 0 | 0 | 5 | 10 | 20 | 40 | 60 | 80 | 100 | 125 | 150 | 200 |
| 340 | 0 | 0 | 10 | 20 | 40 | 60 | 80 | 100 | 125 | 150 | 200 | 250 |
| 375 | 0 | 0 | 20 | 40 | 60 | 80 | 100 | 125 | 150 | 200 | 250 | 300 |
| 400 | 0 | 0 | 20 | 40 | 60 | 80 | 100 | 125 | 150 | 200 | 250 | 300 |
| 425 | 0 | 0 | 40 | 60 | 80 | 100 | 125 | 150 | 200 | 250 | 300 | 350 |
| 450 | 0 | 0 | 40 | 60 | 80 | 100 | 125 | 150 | 200 | 250 | 300 | 350 |

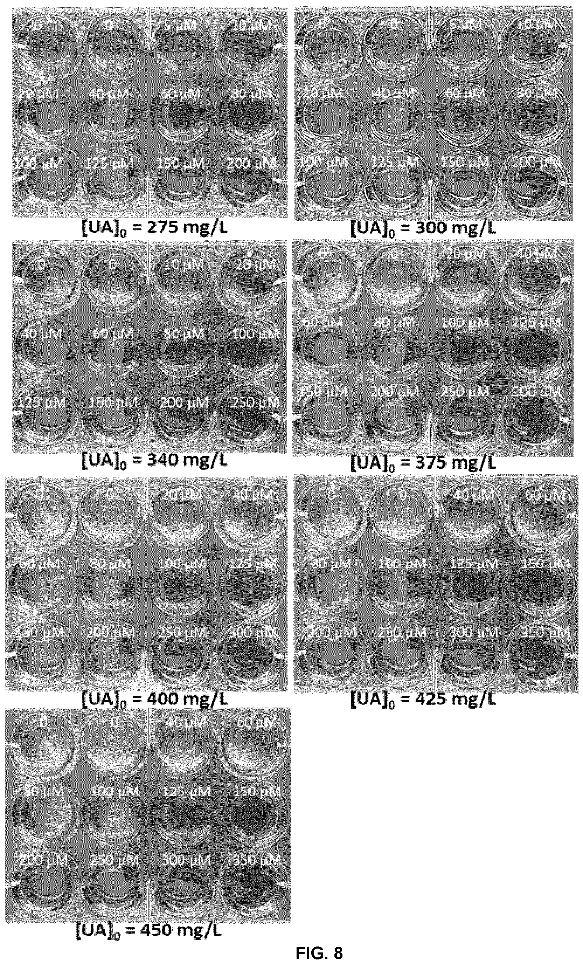

Figure 6, 7, and 9, show the concentration of uric acid in solution measured after 3 days, 4 days, and 5 days, respectively. Figure 8 shows images of the plates after 5 days of incubation.

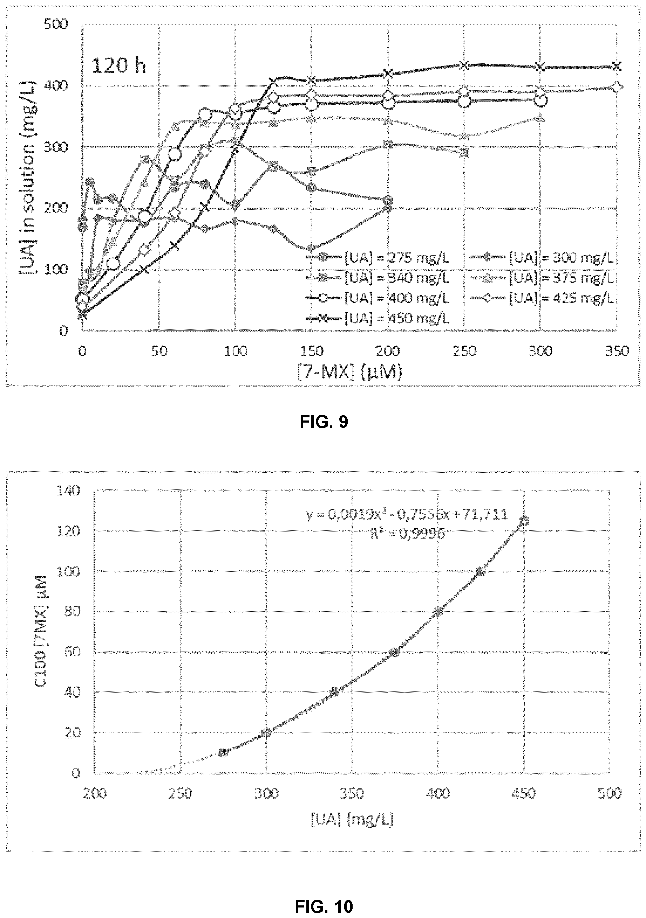

From the data shown in Figures 8 and 9, the minimum concentration of 7-methylxanthine that totally prevented the formation of sodium urate crystals (C100) was determined, as shown in Figure 10 and Table 2:

**Table 2**

| [UA]₀ (mg/L) | C100 |
|---|---|
| 275 | 10 |
| 300 | 20 |
| 340 | 40 |
| 375 | 60 |
| 400 | 80 |
| 425 | 100 |
| 450 | 125 |

These results demonstrate that 7-methylxanthine at low concentrations can prevent the crystallization of sodium urate even when the latter is present in amounts even higher than those that can be found in the interstitial fluid. In particular, the results herein provided show how 7-methylxanthine concentrations of the order of 6 mg/L prevent the formation of sodium urate crystals for times longer than 48h, thus demonstrating the strong effect that 7-methylxanthine can have in the prevention of gout. 3-methylxanthine also showed important inhibitory effects, although for the same concentration its effects were approximately half of those observed with 7-methylxanthine. The effects of 7-methyluric acid were like those of 7-methylxanthine.

Although the results show that caffeine does not affect the crystallization process of sodium urate, it is known that when caffeine is administered orally, 8% of it is metabolized to 7-methylxanthine, and when theobromine (dark chocolate) is administered orally, 30% of it is metabolized to 7-methylxanthine. Therefore, these results show that 7-methylxanthine could be administered directly to subjects, or indirectly in the form of caffeine or theobromine, to treat or prevent gout.

It must be also noted that the increase in the solubility of sodium urate generated by 7-methylxanthine may facilitate its redissolution process, so it could also be important in the treatment of gout attacks.

An important aspect to consider is that 7-methylxanthine has already been used for the treatment of myopia, and its toxicity has been studied, concluding that doses of up to 400 mg three times a day do not present any toxicity. Therefore, 7-methylxanthine is not only an effective treatment against gout, but it is also safe.

Finally, it can be considered that due to the absence of side effects associated with 7-methylxanthine administration, it could also be combined with the administration of drugs that block the production of uric acid, as Allopurinol or Febuxostat, and in such situations the doses of these drugs could be decreased, thus being able to minimize or avoid its side effects.

### Citation List

Arnaud MJ et al., "Pharmacokinetics and Metabolism of Natural Methylxanthines in Animal and Man", Methylxanthines. Handbook of Experimental Pharmacology, vol. 200.

## Claims

1. A compound of formula (I) 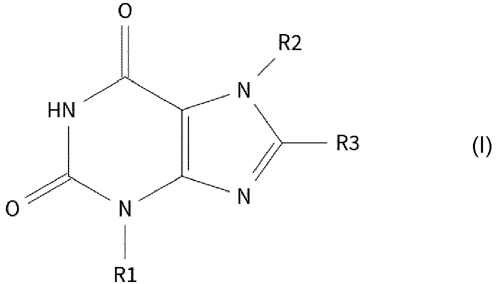
or a pharmaceutically acceptable salt thereof, for use in the prevention and/or treatment of a disease or disorder associated with the crystallization of monosodium urate monohydrate, wherein
R₁ and R₂ are different and are selected from -H and -(C₁-C₆)alkyl; and
R₃ is selected from -H and =O.

2. The compound for use according to claim 1, wherein the disease or disorder associated with the crystallization of monosodium urate monohydrate is gout.

3. The compound for use according to claim 1 or 2, wherein R₁ and R₂ are different and are selected from -H and -CH₃.

4. The compound for use according to any one of claims 1-3, wherein the compound is selected from the group consisting of 7-methylxanthine, 3-methylxanthine, and 7-methyluric acid; particularly the compound is 7-methylxanthine.

5. The compound for use according to any one of claims 1-4, which is comprised in a pharmaceutical composition, a nutraceutical composition, a functional food, or a food supplement.

6. The compound for use according to any one of claim 1-5, which is administered in the form of a metabolic precursor.

7. The compound for use according to claim 6, wherein the metabolic precursor is theobromine or caffeine.

8. The compound for use according to any one of claim 1-7, which is administered in a dose from 1 mg to 4000 mg per day.

9. The compound for use according to claim 8, which is administered in a dose from 500 mg to 1500 mg per day.

10. The compound for use according to any one of claims 1-9, which is administered orally.

11. The compound for use according to any one of claims 1-10, which is administered in combination with a compound selected from the group consisting of colchicine, a compound that inhibits uric acid synthesis, a compound that promotes uric acid excretion, a non-steroidal anti-inflammatory agent, a corticoid, and mixtures thereof.

12. The compound for use according to claim 11, wherein the compound that inhibits uric acid synthesis is Allopurinol or Febuxostat.

13. The compound for use according to claim 11 or 12, wherein the compound that promotes uric acid excretion is selected from the group consisting of Probenecid, Sulfinpyrazone, Benzbromarone and Lesinurad.

14. An *in vitro* method for eliminating and/or preventing the formation of monosodium urate monohydrate crystals in a composition comprising uric acid, the method comprising contacting the composition with a compound of formula (I) 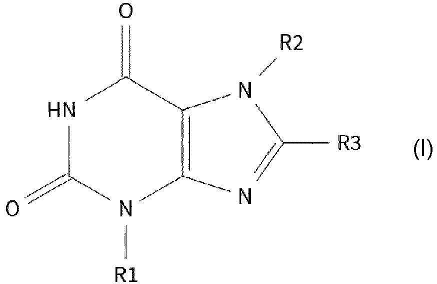 or a pharmaceutically acceptable salt thereof, wherein R₁ and R₂ are different and are selected from -H and -(C₁-C₆)alkyl; and R₃ is selected from -H and =O.

15. *In vitro* use of a compound of formula (I) 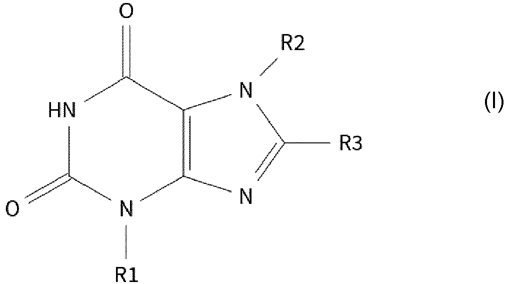 or a pharmaceutically acceptable salt thereof, for eliminating and/or preventing the formation of monosodium urate monohydrate crystals, wherein R₁ and R₂ are different and are selected from -H and -(C₁-C₆)alkyl; and R₃ is selected from -H and =O.
